# EUROPÄISCHE PATENTANMELDUNG

(11) **EP 2 902 477 A1**
(43) Veröffentlichungstag der Anmeldung: **05.08.2015**
(21) Anmeldenummer: 14153036.0
(22) Anmeldetag: 29.01.2014
(51) Int. Cl.: C12M 1/34, C12M 1/00, C12P 1/04, C12P 7/06, C12P 7/08, C12P 7/54

(54) **Erzeugung von C2-C5 Kohlenwasserstoffen mittels Bakterienfermentation**

(71) Anmelder: Siemens VAI Metals Technologies GmbH, 4031 Linz (AT)
(72) Erfinder: Millner, Robert, 3382 Loosdorf (AT); Rosenfellner, Gerald, 3352 Ertl (AT)
(74) Vertreter: Maier, Daniel Oliver

(57) **Zusammenfassung**

Die Erfindung betrifft ein Verfahren und eine Vorrichtung zur Erzeugung von C2-C5 Kohlenwasserstoffen (4) mittels Bakterienfermentation eines Feedgases (5) in einer Fermentationsanlage (2), wobei das Feedgas (5) durch Behandlung eines aus einem Eisenerzeugungsaggregat (8) abgezogenen Exportgases (20) hergestellt wird.

## Beschreibung

### Technisches Gebiet

Die Erfindung betrifft ein Verfahren und eine Vorrichtung zur Erzeugung von C2-C5 Kohlenwasserstoffen mittels Bakterienfermentation eines Feedgases in einer Fermentationsanlage, wobei das Feedgas durch Behandlung eines aus einem Eisenerzeugungsaggregat abgezogenen Exportgases hergestellt wird.

### Stand der Technik

Aus dem Stand der Technik ist bekannt, ein Gas, welches Kohlenmonoxid und Kohlendioxid enthält, zur Herstellung von C2-C5 Kohlenwasserstoffen wie Ethanol, Essigsäure, Isopropanol, n-Butanol, Isopren, Isobutanol, Bernsteinsäure oder Aceton mittels Bakterienfermentation zu verwenden. Ein solches Gas, oftmals auch Feedgas genannt, umfasst beispielsweise ein aus einem Reduktionsaggregat zur Reduktion von Eisenoxid metallischem Eisen abgezogenes Topgas. Auch Koksofengas oder Gas aus einer Kohlevergasungsanlage kommen als Feedgas in Frage. Üblicherweise wird dazu das staubbeladene Feedgas vor dem Einbringen in eine Fermentationsanlage mittels Reinigungseinrichtungen von Staub befreit, gegebenenfalls entspannt, und anschließend bei einem Überdruck von etwa 0,1 bar in einem Niederdruckgasspeicher zwischengespeichert. Als Überdruck wird dabei der Druck über dem Atmosphärendruck bezeichnet. Je nach Bedarf wird das zwischengespeicherte Feedgas aus dem Niederdruckgasspeicher entnommen, anschließend wieder verdichtet, und nach Kühlung in eine Temperature-Pressure-Swing-Adsorption-Anlage (TPSA-Anlage) zur Abtrennung der im Feedgas enthaltenen aromatischen Kohlenwasserstoffe, insbesondere Benzol, Toluol, Ethylbenzol, Xylol (BTEX), eingebracht. Das derart behandelte Feedgas, welches nach seiner Behandlung in der TPSA-Anlage mit Ausnahme der abgetrennten aromatischen Kohlenwasserstoffe im Wesentlichen die gleiche chemische Zusammensetzung aufweist wie vor der Behandlung, wird in der Fermentationsanlage mittels Bakterien nach aus dem Stand der Technik bekannten Reaktionen in Ethanol, Butanol, Aceton beziehungsweise in weitere C2-C5 Kohlenwasserstoffe unter Bildung eines Ventgases umgewandelt. Das bei der Fermentation gebildete Ventgas wird über eine Fackel entsorgt.

Nachteilig dabei ist der in Bezug auf die Fermentation in der Fermentationsanlage relativ niedrige Kohlenmonoxidgehalt beziehungsweise hohe Kohlendioxidgehalt eines derartigen Feedgases. Beispielsweise ist die Menge des aus einer bestimmten Menge des Feedgases herstellbaren Ethanols vom Kohlenmonoxidgehalt im Feedgas abhängig. Je mehr Kohlenmonoxid pro Mengeneinheit des Feedgases enthalten ist, desto mehr Ethanol pro Mengeneinheit des Feedgases kann hergestellt werden, beziehungsweise je mehr Kohlendioxid pro Mengeneinheit des Feedgases enthalten ist, desto weniger Ethanol pro Mengeneinheit des Feedgases kann hergestellt werden. Entsprechendes gilt auch für die anderen genannten C2-C5 Kohlenwasserstoffe.

Ein weiterer Nachteil ergibt sich dadurch, dass das dem Niederdruckgasspeicher entnommene Feedgas vor dem Einbringen in die TPSA-Anlage von einem niedrigen Druckniveau, beispielsweise 0,1 bar Überdruck, auf einen für die Behandlung in der TPSA-Anlage notwendigen Absolutdruck von etwa 6 bar gebracht werden muss. Die dafür notwendige Verdichtung des Feedgases ist mit hohen Anlagenaufwand und Energiekosten verbunden.

Weiter wird in den aus dem Stand der Technik bekannten Verfahren Überschussfeedgas, das ist jener Anteil des Feedgases, welcher der Fermentationsanlage aus kapazitätsgründen nicht zugeführt werden kann, im Allgemeinen einer thermischen Nutzung in einem Kraftwerk unterzogen. Dadurch wird ein im Feedgas enthaltenes Reduktionspotential, welches durch den im Feedgas enthaltenen Kohlenmonoxidgehalt und Wasserstoffgehalt gegeben ist, nicht mehr zur Reduktion des Eisenoxids im Reduktionsaggregat genutzt.

### Zusammenfassung der Erfindung

### Technische Aufgabe

Aufgabe der Erfindung ist die Nutzung eines Exportgases zur Herstellung von C2-C5 Kohlenwasserstoffen mittels Bakterienfermentation unter Vermeidung der aus dem Stand der Technik bekannten Nachteile.

### Technische Lösung

Gelöst wird die Aufgabe durch ein Verfahren zur Erzeugung von C2-C5 Kohlenwasserstoffen mittels Bakterienfermentation eines Feedgases in einer Fermentationsanlage, wobei das Feedgas durch Behandlung zumindest einer Teilmenge eines aus einem ersten Eisenerzeugungsaggregat, aus einem Stahlerzeugungsaggregat, aus einer Kohlevergasungsanlage oder aus einer Koksofengasanlage abgezogenen Exportgases in einer Kohlendioxid-Entfernungseinrichtung hergestellt wird.

Unter C2-C5 Kohlenwasserstoffe sind insbesondere Ethanol, Essigsäure, i-Propanol, Butanediol, n-Butanol, i-Butanol, Aceton und Isopren zu verstehen. Das Feedgas enthält, ebenso wie das Exportgas, zumindest Kohlenmonoxid (CO), Kohlendioxid (CO₂) und Wasserstoff (H₂). Unter Behandlung ist die Abtrennung des Kohlendioxids von zumindest der Teilmenge des Exportgases zu verstehen. Gleichzeitig werden bei der Behandlung zumindest der Teilmenge des Exportgases in der Kohlendioxid-Entfernungseinrichtung der CO-Gehalt angehoben und der H₂-Gehalt verringert. Das derart behandelte Exportgas, beziehungsweise Teilmenge des Exportgases, wird als Feedgas bezeichnet, welches anschließend in die Fermentationsanlage zur Herstellung der C2-C5 Kohlenwasserstoffe eingebracht wird. Unter dem Begriff Eisenerzeugungsaggregat ist eine Vorrichtung zu verstehen, in der eisenoxidhältige Einsatzstoffe mittels eines Reduktionsgases zumindest teilweise zu metallischem Eisen reduziert werden. Unter dem Begriff Stahlerzeugungsaggregat ist eine Vorrichtung zu verstehen, in der Eisen zu Stahl verarbeitet wird. Insbesondere ist unter dem Begriff Eisenerzeugungsaggregat ein Wirbelschichtreduktionsaggregat, eine Wirbelschichtkaskade mit mehreren Wirbelschichtreduktionsaggregaten, ein Hochofen, ein Sauerstoffhochofen, ein Reduktionsschacht beziehungsweise ist unter dem Begriff Stahlerzeugungsaggregat ein Konverter zur Herstellung von Rohstahl zu verstehen.

Das Exportgas, beziehungsweise zumindest die Teilmenge des Exportgases, wird durch die Behandlung in der Kohlendioxid-Entfernungseinrichtung in Bezug auf die Fermentation in der Fermentationsanlage aufgewertet - einerseits durch die Anhebung des Kohlenmonoxidgehalts, andererseits durch die Verringerung des Kohlendioxidgehalts. Die Menge der aus einer bestimmten Menge des Feedgases herstellbaren C2-C5 Kohlenwasserstoffe ist vom Kohlenmonoxidgehalt im Feedgas abhängig. Je mehr Kohlenmonoxid pro Mengeneinheit des Feedgases enthalten ist, desto mehr C2-C5 Kohlenwasserstoffe pro Mengeneinheit des Feedgases können hergestellt werden, beziehungsweise je mehr Kohlendioxid pro Mengeneinheit des Feedgases enthalten ist, desto weniger C2-C5 Kohlenwasserstoffe pro Mengeneinheit des Feedgases können hergestellt werden.

Das aus dem ersten Eisenerzeugungsaggregat abgezogene Exportgas enthält Kohlenmonoxid, Kohlendioxid und Wasserstoff und weist einen Überdruck zwischen 0,5 bar und 3 bar auf. Als Überdruck wird der Druck, über dem Atmosphärendruck bezeichnet. Der für die Behandlung in der Kohlendioxid-Entfernungseinrichtung erforderliche Absolutdruck, das ist der Druck inklusive dem Atmosphärendruck, liegt üblicherweise bei etwa 7 bar. Die entsprechende zu überwindende Druckdifferenz ist im Vergleich zu den aus dem Stand der Technik bekannten Verfahren niedriger - verbunden mit niedrigerem Energieaufwand zur Überwindung dieser Druckdifferenz.

In einer bevorzugten Ausgestaltung des erfindungsgemäßen Verfahrens wird zumindest eine Teilmenge des Feedgases als Reduktionsgas in das erste Eisenerzeugungsaggregat oder in ein zweites Eisenerzeugungsaggregat zur Reduktion von eisenoxidhältigen Einsatzstoffen eingeleitet.

Unter Reduktionsgas ist kohlenmonoxidhältiges und/oder wasserstoffhältiges Gas zu verstehen. Zumindest eine Teilmenge des Feedgases wird als Reduktionsgas in das erste Eisenerzeugungsaggregat oder in das zweite Eisenerzeugungsaggregat eingeleitet. Das zweite Eisenerzeugungsaggregat ist ein vom ersten Eisenerzeugungsaggregat separates Eisenerzeugungsaggregat, beispielsweise ist es, so wie das erste Eisenerzeugungsaggregat, ein Wirbelschichtreduktionsaggregat, eine Wirbelschichtkaskade mit mehreren Wirbelschichtreduktionsaggregaten, ein Hochofen, ein Sauerstoffhochofen oder ein Reduktionsschacht beziehungsweise ein Direktreduktionsschacht.

Durch das Einleiten zumindest der Teilmenge des Feedgases in das erste Eisenerzeugungsaggregat oder in das zweite Eisenerzeugungsaggregat wird jene Menge des in der Kohlendioxid-Entfernungseinrichtung hergestellten Feedgases, welche aus Kapazitätsgründen nicht der Fermentationsanlage zugeführt werden kann - sogenanntes Überschussfeedgas, zur zumindest teilweisen Reduktion der eisenoxidhältigen Einsatzstoffe zu metallischem Eisen genutzt.

Eine andere bevorzugte Ausführungsform des erfindungsgemäßen Verfahren ist dadurch gekennzeichnet, dass zumindest eine Teilmenge des Reduktionsgases vor Einleiten in das erste Eisenerzeugungsaggregat oder in das zweite Eisenerzeugungsaggregat durch Verbrennen eines Brenngases in zumindest einem Reduktionsgasofen auf eine zur Reduktion der eisenoxidhältigen Einsatzstoffe erforderliche Temperatur aufgeheizt wird und dass das Brenngas ein bei der Bakterienfermentation in der Fermentationsanlage anfallendes Ventgas umfasst.

Im Reduktionsgasofen wird zumindest die Teilmenge des Reduktionsgases vor dem Einleiten in das erste Eisenerzeugungsaggregat oder in das zweite Eisenerzeugungsaggregat auf eine zur Reduktion der eisenoxidhältigen Einsatzstoffe erforderliche Temperatur aufgeheizt. Der Reduktionsgasofen kann dabei auch zweistufig ausgeführt sein. In der ersten Stufe des Reduktionsgasofens wird zumindest die Teilmenge des Reduktionsgases auf etwa 450°C aufgeheizt, in der zweiten Stufe des Reduktionsgasofens erfolgt eine aus dem Stand der Technik bekannte partielle Oxidation zumindest der Teilmenge des Reduktionsgases mit Sauerstoff. Dabei wird der ersten Stufe des Reduktionsgasofens das Ventgas als Brenngas zugeführt. Unter Ventgas ist ein Gas zu verstehen, das bei der Bakterienfermentation des Feedgases in der Fermentationsanlage anfällt.
Damit kann das Ventgas innerhalb des erfindungsgemäßen Verfahrens wirtschaftlich genutzt werden und muss nicht mit hohem Aufwand, beispielsweise über Fackeln, entsorgt werden.

In einer Ausführungsform des erfindungsgemäßen Verfahrens wird zumindest eine Teilmenge des Ventgases zur thermischen Nutzung in ein Kraftwerk eingeleitet.

Damit kann das Ventgas nicht nur als Brenngas im Reduktionsgasofen genutzt werden, sondern auch in andere nutzbare Energieformen umgewandelt werden.

In einer weiteren Ausführungsform des erfindungsgemäßen Verfahrens beträgt der Kohlenmonoxidgehalt des Feedgases mindestens 35 Volumsprozent, bevorzugt mindestens 45 Volumsprozent, besonders bevorzugt mindestens 50 Volumsprozent.

Durch den hohen Kohlenmonoxidgehalt im Feedgas wird die Effizienz der Bakterienfermentation in der Fermentationsanlage im Vergleich zu aus dem Stand der Technik bekannten Verfahren erhöht.

Der Kohlendioxidgehalt des Feedgases beträgt maximal 8 Volumsprozent, bevorzugt maximal 5 Volumsprozent, besondersbevorzugt maximal 3 Volumsprozent.

Durch den niedrigen Kohlendioxidgehalt im Feedgas wird die Effizienz der Bakterienfermentation in der Fermentationsanlage im Vergleich zu aus dem Stand der Technik bekannten Verfahren erhöht.

Das Exportgas weist zwischen 25 Volumsprozent und 50 Volumsprozent Kohlenmonoxid, zwischen 25 Volumsprozent und 45 Volumsprozent Kohlendioxid und zwischen 8 Volumsprozent und 25 Volumsprozent Wasserstoff auf.

In einer anderen bevorzugten Ausführungsform des erfindungsgemäßen Verfahrens handelt es sich bei der Kohlendioxid-Entfernungseinrichtung um eine PSA-Anlage oder um eine VPSA-Anlage.

Mittels der PSA-Anlage oder der VPSA-Anlage, welche aus dem Stand der Technik hinreichend bekannt sind, wird Kohlendioxid aus zumindest der Teilmenge des Exportgases unter Bildung des Feedgases abgetrennt. Gleichzeitig werden mit dem Kohlendioxid auch aromatische Kohlenwasserstoffe, welche sich negativ auf die Bakterienfermentation in der Fermentationsanlage auswirken, vom Exportgas, beziehungsweise von zumindest der Teilmenge des Exportgases, abgetrennt. Einerseits wird dadurch ein hochqualitatives Feedgas zur Erzeugung der C2-C5 Kohlenwasserstoffe hergestellt, andererseits wird ein hochqualitatives Reduktionsgas mit einem hohen Kohlenmonoxidgehalt zur Reduktion der eisenoxidhältigen Einsatzstoffe im ersten oder im zweiten Eisenerzeugungsaggregat bereitgestellt.

Durch die Kombination der PSA-Anlage beziehungsweise der VPSA-Anlage mit der Fermentationsanlage, beziehungsweise mit dem ersten oder dem zweiten Eisenerzeugungsaggregat ergibt sich ein synergetischer Effekt, der sich in einer höheren Wirtschaftlichkeit - sowohl in Bezug auf die Reduktion als auch in Bezug auf die Bakterienfermentation, widerspiegelt. Zusätzlich werden dadurch die Investitionskosten gesenkt.

Ein bei der Behandlung zumindest der Teilmenge des Exportgases in der Kohlendioxid-Entfernungseinrichtung anfallendes Tailgas wird in einer weiteren Ausführungsform des erfindungsgemäßen Verfahrens im Reduktionsgasofen und/oder im Kraftwerk thermisch verwertet.

Dadurch wird die Wirtschaftlichkeit des erfindungsgemäßen Verfahrens durch Nutzung des Tailgases als Brenngas im Reduktionsgasofen beziehungsweise durch thermische Nutzung im Kraftwerk weiter erhöht.

Der Sauerstoffgehalt des Feedgases beträgt maximal 100 ppm, bevorzugt maximal 50 ppm, besonders bevorzugt maximal 10 ppm.

Unter ppm ist parts per million zu verstehen. Ein zu hoher Sauerstoffgehalt im Feedgas wirkt sich negativ auf die Bakterienfermentation in der Fermentationsanlage aus. Auf Grund des niedrigen Sauerstoffgehaltes im Feedgas kann das Feedgas, ohne vorherige Umsetzung des in ihm enthaltenen Sauerstoffs, der Fermentationsanlage zugeführt werden.

Ein weiterer Gegenstand der vorliegenden Anmeldung ist eine Vorrichtung zur Durchführung des erfindungsgemäßen Verfahrens, wobei die Vorrichtung umfasst:
- eine Fermentationsanlage mit einer Feedgaszufuhrleitung zur Erzeugung von C2-C5 Kohlenwasserstoffen mittels Bakterienfermentation eines Feedgases,
- eine Kohlendioxid-Entfernungseinrichtung mit einer Feedgasabfuhrleitung, welche mit der Feedgaszufuhrleitung verbunden ist und
- ein erstes Eisenerzeugungsaggregat, ein Stahlerzeugungsaggregat, eine Kohlevergasungsanlage oder eine Koksofengasanlage mit einer Exportgasabfuhrleitung, welche in die Kohlendioxid-Entfernungseinrichtung mündet.

Mittels der Feedgaszufuhrleitung kann der Fermentationsanlage zumindest eine Teilmenge des in der Kohlendioxid-Entfernungseinrichtung hergestellten Feedgases zugeführt werden. Das erste Eisenerzeugungsaggregat, das Stahlerzeugungsaggregat, die Kohlevergasungsanlage oder die Koksofengasanlage verfügen über eine Exportgasabfuhrleitung zur Abfuhr von darin anfallendem Exportgas. Das Exportgas, beziehungsweise zumindest eine Teilmenge des Exportgases, kann mittels der Exportgasabfuhrleitung in die Kohlendioxid-Entfernungsanlage eingebracht werden.

Das Exportgas, beziehungsweise zumindest die Teilmenge des Exportgases, wird durch die Behandlung in der Kohlendioxid-Entfernungseinrichtung in Bezug auf die Fermentation in der Fermentationsanlage aufgewertet - einerseits durch die Anhebung des Kohlenmonoxidgehalts, andererseits durch die Verringerung des Kohlendioxidgehalts. Die Menge der aus einer bestimmten Menge des Feedgases herstellbaren C2-C5 Kohlenwasserstoffe ist vom Kohlenmonoxidgehalt im Feedgas abhängig. Je mehr Kohlenmonoxid pro Mengeneinheit des Feedgases enthalten ist, desto mehr C2-C5 Kohlenwasserstoffe pro Mengeneinheit des Feedgases können hergestellt werden, beziehungsweise je mehr Kohlendioxid pro Mengeneinheit des Feedgases enthalten ist, desto weniger C2-C5 Kohlenwasserstoffe pro Mengeneinheit des Feedgases können hergestellt werden.

Das aus dem ersten Eisenerzeugungsaggregat, aus dem Stahlerzeugungsaggregat, aus der Kohlevergasungsanlage oder aus der Koksofengasanlage abgezogene Exportgas enthält Kohlenmonoxid, Kohlendioxid und Wasserstoff und weist einen Überdruck zwischen 0,5 bar und 3 bar auf. Der für die Behandlung in der Kohlendioxid-Entfernungseinrichtung erforderliche Absolutdruck liegt üblicherweise bei etwa 6 bar. Die entsprechende zu überwindende Druckdifferenz ist im Vergleich zu den aus dem Stand der Technik bekannten Verfahren niedriger - verbunden mit niedrigerem Energieaufwand zur Überwindung dieser Druckdifferenz.

Ein bevorzugte Ausführungsform der erfindungsgemäßen Vorrichtung ist dadurch gekennzeichnet, dass die Feedgasabfuhrleitung mit einer Recyclegasleitung verbunden ist, welche mit einer in das erste Eisenerzeugungsaggregat mündenden ersten Reduktionsgaszufuhrleitung verbunden ist oder dass ein zweites Eisenerzeugungsaggregat mit einer zweiten Reduktionsgaszufuhrleitung vorhanden ist, wobei die Feedgasabfuhrleitung mit der zweiten Reduktionsgaszufuhrleitung verbunden ist.

Zumindest eine Teilmenge des Feedgases wird mittels der Recyclegasleitung und der ersten Reduktionsgaszufuhrleitung beziehungsweise mittels der zweiten Reduktionsgaszufuhrleitung als Reduktionsgas in das erste Eisenerzeugungsaggregat beziehungsweise in das zweite Eisenerzeugungsaggregat eingeleitet. Das zweite Eisenerzeugungsaggregat ist ein vom ersten Eisenerzeugungsaggregat separates Eisenerzeugungsaggregat, beispielsweise ist es, so wie das erste Eisenerzeugungsaggregat, ein Wirbelschichtreduktionsaggregat, eine Wirbelschichtkaskade mit mehreren Wirbelschichtreduktionsaggregaten, ein Hochofen, ein Sauerstoffhochofen oder ein Reduktionsschacht beziehungsweise ein Direktreduktionsschacht.

Durch das Einleiten der Teilmenge des Feedgases in das erste Eisenerzeugungsaggregat oder in das zweite Eisenerzeugungsaggregat wird jene Menge des in der Kohlendioxid-Entfernungseinrichtung hergestellten Feedgases, welche aus Kapazitätsgründen nicht der Fermentationsanlage zugeführt werden kann - sogenanntes Überschussfeedgas, zur Reduktion der eisenoxidhältigen Einsatzstoffe zu metallischem Eisen genutzt.

In einer weiteren bevorzugten Ausführungsform der erfindungsgemäßen Vorrichtung ist in der Recyclegasleitung oder in der zweiten Reduktionsgaszufuhrleitung ein Reduktionsgasofen mit einer Ventgasleitung zur Zufuhr eines Ventgases, welches bei der Bakterienfermentation des Feedgases anfällt, angeordnet, wobei die Ventgasleitung mit der Fermentationsanlage verbunden ist.

Zumindest eine Teilmenge des bei der Bakterienfermentation in der Fermentationsanlage anfallenden Ventgases kann dem Reduktionsgasofen mittels der Ventgasleitung zugeführt werden. Im Reduktionsgasofen wird das Reduktionsgas vor dem Einleiten in das erste Eisenerzeugungsaggregat oder in das zweite Eisenerzeugungsaggregat auf eine zur Reduktion der eisenoxidhältigen Einsatzstoffe erforderliche Temperatur aufgeheizt. Der Reduktionsgasofen kann dabei auch zweistufig ausgeführt sein. In der ersten Stufe des Reduktionsgasofens wird das Reduktionsgas auf etwa 450°C aufgeheizt, in der zweiten Stufe des Reduktionsgasofens erfolgt eine aus dem Stand der Technik bekannte partielle Oxidation des Reduktionsgases mit Sauerstoff. Dabei wird der ersten Stufe des Reduktionsgasofens das Ventgas als Brenngas zugeführt. Unter Ventgas ist ein Gas zu verstehen, das bei der Bakterienfermentation des Feedgases in der Fermentationsanlage anfällt.
Damit kann das Ventgas innerhalb des erfindungsgemäßen Verfahrens und innerhalb der erfindungsgemäßen Vorrichtung wirtschaftlich genutzt werden und muss nicht mit hohem Aufwand, beispielsweise über Fackeln, entsorgt werden.

Eine weitere Ausführungsform der erfindungsgemäßen Vorrichtung ist dadurch gekennzeichnet, dass die Vorrichtung ein Kraftwerk umfasst, welches mittels einer Ventgaszufuhrleitung mit der Fermentationsanlage verbunden ist.

Dem Kraftwerk kann zumindest eine Teilmenge des bei der Bakterienfermentation anfallenden Ventgases mittels der Ventgaszufuhrleitung zugeführt werden.

Damit kann das Ventgas nicht nur als Brenngas im Reduktionsgasofen genutzt werden, sondern im Kraftwerk auch in andere nutzbare Energieformen umgewandelt werden.

In einer anderen Ausführungsform der erfindungsgemäßen Vorrichtung ist die Kohlendioxid-Entfernungseinrichtung als PSA-Anlage oder als VPSA-Anlage ausgebildet.

Mittels der PSA-Anlage oder der VPSA-Anlage, welche aus dem Stand der Technik hinreichend bekannt sind, wird Kohlendioxid vom Exportgas, beziehungsweise von zumindest der Teilmenge des Exportgases, unter Bildung des Feedgases abgetrennt. Gleichzeitig werden mit dem Kohlendioxid auch aromatische Kohlenwasserstoffe, welche sich negativ auf die Bakterienfermentation in der Fermentationsanlage auswirken, vom Exportgas, beziehungsweise von zumindest der Teilmenge des Exportgases, abgetrennt. Einerseits wird dadurch ein hochqualitatives Feedgas zur Erzeugung der C2-C5 Kohlenwasserstoffe hergestellt, andererseits wird ein hochqualitatives Reduktionsgas mit einem hohen Kohlenmonoxidgehalt zur zumindest teilweisen Reduktion der eisenoxidhältigen Einsatzstoffe im ersten oder zweiten Eisenerzeugungsaggregat bereitgestellt.

Durch die Kombination einer PSA-Anlage beziehungsweise VPSA-Anlage mit der Fermentationsanlage, beziehungsweise mit dem ersten oder zweiten Eisenerzeugungsaggregat ergibt sich ein synergetischer Effekt, der sich in einer höheren Wirtschaftlichkeit - sowohl in Bezug auf die zumindest teilweise Reduktion als auch in Bezug auf die Bakterienfermentation, widerspiegelt. Zusätzlich werden dadurch die Investitionskosten gesenkt.

Die Kohlendioxid-Entfernungseinrichtung ist in einer weiteren Ausführungsform der erfindungsgemäßen Vorrichtung mittels einer Tailgasleitung mit dem Kraftwerk und/oder mit dem Reduktionsgasofen verbunden ist.

Dadurch wird die Wirtschaftlichkeit des erfindungsgemäßen Verfahrens durch Nutzung des Tailgases als Brenngas im Reduktionsgasofen beziehungsweise durch thermische Nutzung im Kraftwerk weiter erhöht.

### Kurze Beschreibung der Zeichnungen

FIG 1 zeigt beispielhaft und schematisch ein erfindungsgemäßes Verfahren und eine erfindungsgemäße Vorrichtung mit einer COREX® - Anlage 1.
FIG 2 zeigt beispielhaft und schematisch ein erfindungsgemäßes Verfahren und eine erfindungsgemäße Vorrichtung mit einer FINEX® - Anlage 21.
FIG 3 zeigt beispielhaft und schematisch ein erfindungsgemäßes Verfahren und eine erfindungsgemäße Vorrichtung mit einem Direktreduktionsschacht.

### Beschreibung der Ausführungsformen

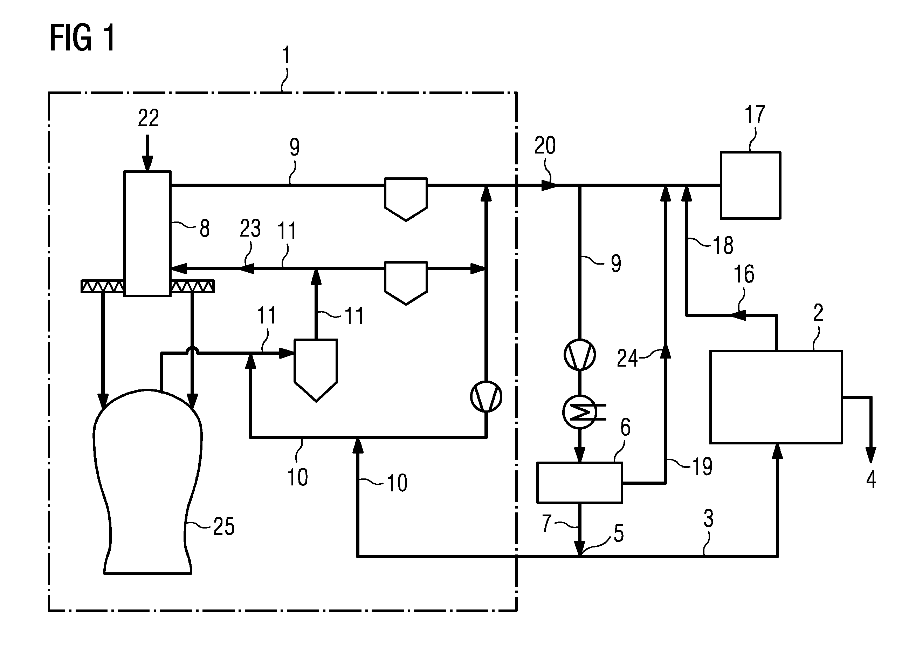

FIG 1 zeigt das erfindungsgemäße Verfahren und die erfindungsgemäße Vorrichtung mit einer COREX® - Anlage 1. Dabei werden C2-C5 Kohlenwasserstoffe 4 mittels Bakterienfermentation eines Feedgases 5 in einer Fermentationsanlage 2 erzeugt. Das Feedgas 5 wird durch Behandlung eines mittels einer Exportgasabfuhrleitung 9 aus einem ersten Eisenerzeugungsaggregat 8, konkret aus einem Reduktionsschacht, abgezogenen Exportgases 20, beziehungsweise einer Teilmenge des Exportgases 20, in einer Kohlendioxid-Entfernungseinrichtung 6 hergestellt, wobei die Exportgasabfuhrleitung 9 in die Kohlendioxid-Entfernungseinrichtung 6 mündet. Der Fermentationsanlage 2 wird das Feedgas 5 mittels einer Feedgaszufuhrleitung 3 zugeführt. Die Kohlendioxid-Entfernungseinrichtung 6 umfasst eine Feedgasabfuhrleitung 7, welche mit der Feedgaszufuhrleitung 3 verbunden ist. Das erste Eisenerzeugungsaggregat 8 ist Teil der COREX® - Anlage 1. Im ersten Eisenerzeugungsaggregat 8 werden eisenoxidhältige Einsatzstoffe 22 mittels eines Reduktionsgases 23, zumindest teilweise, zu metallischem Eisen reduziert. Die zumindest teilweise reduzierten eisenoxidhältigen Einsatzstoffe 22 werden anschließend, unter Zugabe eines sauerstoffhältigen Gases und Kohlenstoffträger, im Einschmelzvergaser 25 fertigreduziert und zu flüssigem Roheisen geschmolzen. Dem ersten Eisenerzeugungsaggregat 8 wird ein dabei gebildetes Reduktionsgas 23, nach Abkühlung und Reinigung, mittels einer ersten Reduktionsgaszufuhrleitung 11 zugeführt.

Mittels einer Recyclegasleitung 10, welche mit der Feedgasabfuhrleitung 7 verbunden ist und welche in die erste Reduktionsgaszufuhrleitung 11 mündet, wird zumindest eine Teilmenge des Feedgases 5 als Reduktionsgas 23 in das erste Eisenerzeugungsaggregat 8 eingebracht.

In einer in FIG 1 aus Übersichtlichkeitsgründen nicht dargestellten Variante wird zumindest eine Teilmenge des Reduktionsgases 23, vor Einleiten in das erste Eisenerzeugungsaggregat 8, mittels eines Redukionsgasofens auf eine zur Reduktion der eisenoxidhältigen Einsatzstoffe 22 erforderliche Temperatur aufgeheizt. Die dafür erforderliche Wärme wird durch Verbrennen eines Brenngases, welches ein bei der Bakterienfermentation in der Fermentationsanlage 2 anfallendes Ventgas 16 umfasst, generiert.

Zumindest eine Teilmenge des Ventgases 16 wird in ein Kraftwerk 17 eingeleitet, welches mittels Ventgaszufuhrleitung 18 mit der Fermentationsanlage 2 verbunden ist.

Ein bei der Behandlung zumindest der Teilmenge des Exportgases 20 in der Kohlendioxid-Entfernungseinrichtung 6 anfallendes Tailgas 24 wird im Kraftwerk 17 oder in dem in FIG 1 nicht dargestellten Reduktionsgasofen thermisch verwertet, wobei die Kohlendioxid-Entfernungseinrichtung 6 mittels einer Tailgasleitung 19 mit dem Kraftwerk 17 verbunden ist.

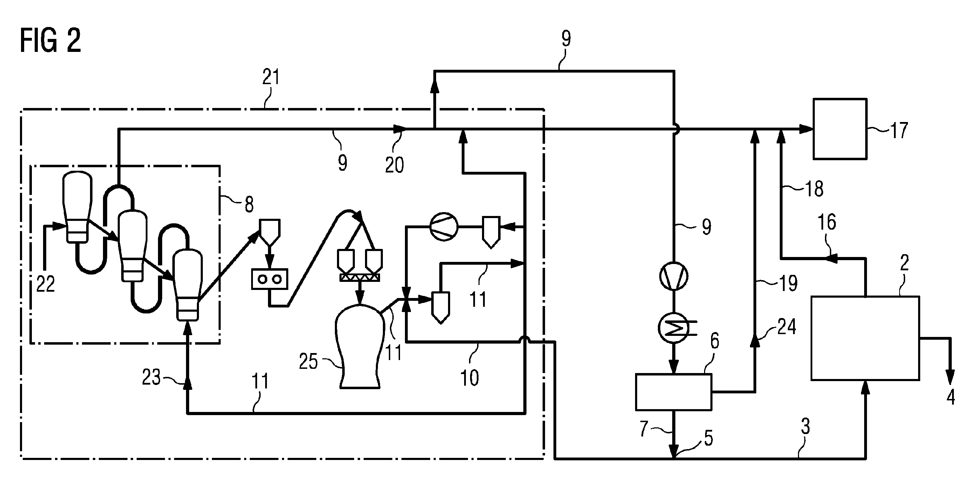

FIG 2 zeigt das erfindungsgemäße Verfahren und die erfindungsgemäße Vorrichtung mit der FINEX® - Anlage 21. Das in FIG 2 dargestellte Verfahren unterscheidet sich vom in FIG 1 dargestellten Verfahren im Wesentlichen dadurch, dass das erste Eisenerzeugungsaggregat 8 drei Wirbelschichtreduktionsaggregate, welche in einer Wirbelschichtkaskade angeordnet sind, umfasst. Dabei durchströmt das Reduktionsgas 23 die einzelnen Wirbelschichtreduktionsaggregate im Gegenstrom zu den eisenoxidhältigen Einsatzstoffen 22. Die dabei zumindest teilweise zu metallischem Eisen reduzierten eisenoxidhältigen Einsatzstoffe 22 werden agglomeriert und anschließend im Einschmelzvergaser 25 unter Zugabe eines sauerstoffhältigen Gases und Kohlenstoffträger fertigreduziert und zu Roheisen geschmolzen. Nach dem das Reduktionsgas 23 - in Strömungsrichtung des Reduktionsgases 23 gesehen - das letzte Wirbelschichtreduktionsaggregat durchströmt hat, wird es mittels Exportgasleitung 9 als Exportgas 20 aus diesem abgezogen, wobei zumindest eine Teilmenge des Exportgases 20 in die Kohlendioxid-Entfernungseinrichtung 6 eingeleitet wird. Die Herstellung und Nutzung des Feedgases 5 erfolgt gemäß dem in FIG 1 beschriebenen Verfahren, wobei die Bezugszeichen der FIG 2 den Bezugszeichen der FIG 1 entsprechen.

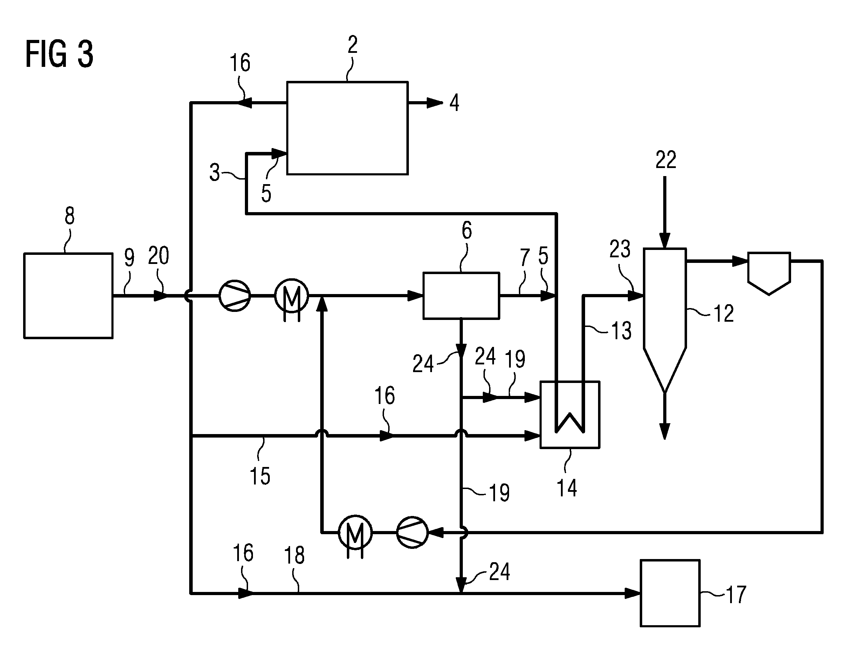

FIG 3 zeigt das erfindungsgemäße Verfahren und die erfindungsgemäße Vorrichtung mit dem Direktreduktionsschacht zur Herstellung der C2-C5 Kohlenwasserstoffe 4.

Das aus dem ersten Eisenerzeugungsaggregat 8 abgezogene Exportgas 20, beziehungsweise zumindest eine Teilmenge des abgezogenen Exportgases 20, wird mittels der Exportgasabfuhrleitung 9, gegebenenfalls nach Verdichtung und Abkühlung, in die Kohlendioxid-Entfernungseinrichtung 6 eingeleitet. Das erste Eisenerzeugungsaggregat 8 ist beispielsweise als Wirbelschichtreduktionsaggregat, als Wirbelschichtkaskade mit mehreren Wirbelschichtreduktionsaggregaten oder als Hochofen ausgebildet. Anstelle des ersten Eisenerzeugungsaggregates 8 kann auch ein Stahlerzeugungsaggregat, eine Kohlevergasungsanlage oder eine Koksofengasanlage vorhanden sein, aus welchem das Exportgas 20 abgezogen wird. Das Feedgas 5 wird durch Behandlung des mittels der Exportgasabfuhrleitung 9 aus dem ersten Eisenerzeugungsaggregat 8 abgezogenen Exportgases 20, beziehungsweise der Teilmenge des Exportgases 20, in der Kohlendioxid-Entfernungseinrichtung 6 hergestellt. Der Fermentationsanlage 2 wird das Feedgas 5, beziehungsweise zumindest eine Teilmenge des Feedgases 5, mittels einer Feedgaszufuhrleitung 3 zugeführt. Die Kohlendioxid-Entfernungseinrichtung 6 umfasst eine Feedgasabfuhrleitung 7, welche mit der Feedgaszufuhrleitung 3 verbunden ist. Zumindest eine weitere Teilmenge des Feedgases 5 wird mittels einer zweiten Reduktionsgaszufuhrleitung 13 als Reduktionsgas 23 in ein zweites Eisenerzeugungsaggregat 12, im in FIG 3 dargestellten Fall in einen Direktreduktionsschacht, eingeleitet. Dazu ist die Feedgasabfuhrleitung 7 mit der zweiten Reduktionsgaszufuhrleitung 13 verbunden. Mittels des Reduktionsgases 23 erfolgt zumindest eine teilweise Reduktion von in den Direktreduktionsschacht eingebrachten eisenoxidhältigen Einsatzstoffen 22 zu metallischem Eisen, beziehungsweise Eisenschwamm. Aus dem Redukionsschacht abgezogenes Topgas wird, gegebenenfalls nach Reinigung und/oder Verdichtung und/oder Abkühlung wieder in die Exportgasabfuhrleitung 9 eingebracht.

Das Reduktionsgas 23 wird vor dem Einleiten in das zweite Eisenerzeugungsaggregat 8 mittels des in der zweiten Reduktionsgaszufuhrleitung 13 angeordneten Reduktionsgasofens 14 auf eine zur Reduktion der eisenoxidhältigen Einsatzstoffe 22 erforderliche Temperatur aufgeheizt. Die dafür erforderliche Wärme wird durch Verbrennen eines Brenngases, welches ein bei der Bakterienfermentation in der Fermentationsanlage 2 anfallendes Ventgas 16 umfasst, generiert. Das Ventgas 16 wird dem Reduktionsgasofen 14 mittels einer Ventgasleitung 15 zugeführt, welche mit der Fermentationsanlage 2 verbunden ist. In einer in FIG 3 aus Übersichtlichkeitsgründen nicht dargestellten Variante des erfindungsgemäßen Verfahrens beziehungsweise Vorrichtung wird eine Teilmenge des aus dem reduktionsschacht abgezogenen Topgases als Brenngas im Reduktionsgasofen 14 genutzt. Zumindest eine Teilmenge des Ventgases 16 wird in das Kraftwerk 17 eingeleitet, welches mittels der Ventgaszufuhrleitung 18 mit der Fermentationsanlage 2 verbunden ist. Gegebenenfalls wird in einer weiteren, aus Übersichtlichkeitsgründen in FIG 3 nicht dargestellten Variante des erfindungsgemäßen Verfahrens beziehungsweise Vorrichtung, zum Halten des Druckes in der Ventgaszufuhrleitung 18, eine Teilmenge des abgezogenen Topgases vor der Verdichtung in die Ventgaszufuhrleitung 18 beziehungsweise in das Kraftwerk 17 eingeleitet.

Das bei der Behandlung zumindest der Teilmenge des Exportgases 20 in der Kohlendioxid-Entfernungseinrichtung 6 anfallende Tailgas 24 wird im Kraftwerk 17 oder im Reduktionsgasofen 14 thermisch verwertet, wobei die Kohlendioxid-Entfernungseinrichtung 6 mittels einer Tailgasleitung 19 mit dem Kraftwerk 17 und mit dem Reduktionsgasofen 14 verbunden ist.

Obwohl die Erfindung im Detail durch die bevorzugten Ausführungsbeispiele näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

### Liste der Bezugszeichen

- 1): COREX® - Anlage
- 2): Fermentationsanlage
- 3): Feedgaszufuhrleitung
- 4): C2-C5 Kohlenwasserstoffe
- 5): Feedgas
- 6): Kohlendioxid-Entfernungseinrichtung
- 7): Feedgasabfuhrleitung
- 8): erstes Eisenerzeugungsaggregat
- 9): Exportgasabfuhrleitung
- 10): Recyclegasleitung
- 11): erste Reduktionsgaszufuhrleitung
- 12): zweites Eisenerzeugungsaggregat
- 13): zweite Reduktionsgaszufuhrleitung
- 14): Reduktionsgasofen
- 15): Ventgasleitung
- 16): Ventgas
- 17): Kraftwerk
- 18): Ventgaszufuhrleitung
- 19): Tailgasleitung
- 20): Exportgas
- 21): FINEX® - Anlage
- 22): eisenoxidhältige Einsatzstoffe
- 23): Reduktionsgas
- 24): Tailgas
- 25): Einschmelzvergaser

## Patentansprüche

1. Verfahren zur Erzeugung von C2-C5 Kohlenwasserstoffen (4) mittels Bakterienfermentation eines Feedgases (5) in einer Fermentationsanlage (2), **dadurch gekennzeichnet, dass** das Feedgas (5) durch Behandlung zumindest einer Teilmenge eines aus einem ersten Eisenerzeugungsaggregat (8), aus einem Stahlerzeugungsaggregat, aus einer Kohlevergasungsanlage oder aus einer Koksofengasanlage abgezogenen Exportgases (20) in einer Kohlendioxid-Entfernungseinrichtung (6) hergestellt wird.

2. Verfahren nach Anspruch 1, **dadurch gekennzeichnet, dass** zumindest eine Teilmenge des Feedgases (5) als Reduktionsgas (23) in das erste Eisenerzeugungsaggregat (8) oder in ein zweites Eisenerzeugungsaggregat (12) zur Reduktion von eisenoxidhältigen Einsatzstoffen (22) eingeleitet wird.

3. Verfahren nach Anspruch 2, **dadurch gekennzeichnet, dass** zumindest eine Teilmenge des Reduktionsgases (23) vor Einleiten in das erste Eisenerzeugungsaggregat (8) oder in das zweite Eisenerzeugungsaggregat (12) durch Verbrennen eines Brenngases in zumindest einem Reduktionsgasofen (14) auf eine zur Reduktion der eisenoxidhältigen Einsatzstoffe (22) erforderliche Temperatur aufgeheizt wird und dass das Brenngas ein bei der Bakterienfermentation in der Fermentationsanlage (2) anfallendes Ventgas (16) umfasst.

4. Verfahren nach Anspruch 3, **dadurch gekennzeichnet, dass** zumindest eine Teilmenge des Ventgases (16) zur thermischen Nutzung in ein Kraftwerk (17) eingeleitet wird.

5. Verfahren nach einem der vorhergehenden Ansprüche, **dadurch gekennzeichnet, dass** der Kohlenmonoxidgehalt des Feedgases (5) mindestens 35 Volumsprozent, bevorzugt mindestens 45 Volumsprozent, besonders bevorzugt mindestens 50 Volumsprozent beträgt.

6. Verfahren nach einem der vorhergehenden Ansprüche, **dadurch gekennzeichnet, dass** der Kohlendioxidgehalt des Feedgases (5) maximal 8 Volumsprozent, bevorzugt maximal 5 Volumsprozent, besonders bevorzugt maximal 3 Volumsprozent beträgt.

7. Verfahren nach einem der vorhergehenden Ansprüche, **dadurch gekennzeichnet, dass** das Exportgas (20) zwischen 25 Volumsprozent und 50 Volumsprozent Kohlenmonoxid, zwischen 25 Volumsprozent und 45 Volumsprozent Kohlendioxid und zwischen 8 Volumsprozent und 25 Volumsprozent Wasserstoff umfasst.

8. Verfahren nach einem der vorhergehenden Ansprüche, **dadurch gekennzeichnet, dass** es sich bei der Kohlendioxid-Entfernungseinrichtung (6) um eine PSA-Anlage oder um eine VPSA-Anlage handelt.

9. Verfahren nach einem der Ansprüche 3 bis 10, **dadurch gekennzeichnet, dass** ein bei der Behandlung zumindest der Teilmenge des Exportgases (20) in der Kohlendioxid-Entfernungseinrichtung (6) anfallendes Tailgas (24) im Reduktionsgasofen (14) und/oder im Kraftwerk (17) thermisch verwertet wird.

10. Verfahren nach einem der vorhergehenden Ansprüche, **dadurch gekennzeichnet, dass** der Sauerstoffgehalt des Feedgases (5) maximal 100 ppm, bevorzugt maximal 50 ppm, besonders bevorzugt maximal 10 ppm beträgt.

11. Vorrichtung zur Durchführung des Verfahrens nach einem der Ansprüche 1 bis 10, umfassend:
• eine Fermentationsanlage (2) mit einer Feedgaszufuhrleitung (3) zur Erzeugung von C2-C5 Kohlenwasserstoffen (4) mittels Bakterienfermentation eines Feedgases (5),
• eine Kohlendioxid-Entfernungseinrichtung (6) mit einer Feedgasabfuhrleitung (7), welche mit der Feedgaszufuhrleitung (3) verbunden ist und
• ein erstes Eisenerzeugungsaggregat (8), ein Stahlerzeugungsaggregat, eine Kohlevergasungsanlage oder eine Koksofengasanlage mit einer Exportgasabfuhrleitung (9), welche in die Kohlendioxid-Entfernungseinrichtung (6) mündet.

12. Vorrichtung nach Anspruch 11, **dadurch gekennzeichnet, dass** die Feedgasabfuhrleitung (7) mit einer Recyclegasleitung (10) verbunden ist, welche mit einer in das erste Eisenerzeugungsaggregat (8) mündenden ersten Reduktionsgaszufuhrleitung (11) verbunden ist oder dass ein zweites Eisenerzeugungsaggregat (12) mit einer zweiten Reduktionsgaszufuhrleitung (13) vorhanden ist, wobei die Feedgasabfuhrleitung (7) mit der zweiten Reduktionsgaszufuhrleitung (13) verbunden ist.

13. Vorrichtung nach Anspruch 12, **dadurch gekennzeichnet, dass** in der Recyclegasleitung (10) oder in der zweiten Reduktionsgaszufuhrleitung (13) ein Reduktionsgasofen (14) mit einer Ventgasleitung (15) zur Zufuhr eines Ventgases (16), welches bei der Bakterienfermentation des Feedgases (5) anfällt, angeordnet ist, wobei die Ventgasleitung (15) mit der Fermentationsanlage (2) verbunden ist.

14. Vorrichtung nach Anspruch 13, **dadurch gekennzeichnet, dass** die Vorrichtung ein Kraftwerk (17) umfasst, welches mittels einer Ventgaszufuhrleitung (18) zur Zufuhr des Ventgases (16) mit der Fermentationsanlage (2) verbunden ist.

15. Vorrichtung nach einem der Ansprüche 11 bis 14, **dadurch gekennzeichnet, dass** die Kohlendioxid-Entfernungseinrichtung (6) eine PSA-Anlage oder eine VPSA-Anlage ist.

16. Vorrichtung nach einem der Ansprüche 14 oder 15, **dadurch gekennzeichnet, dass** die Kohlendioxid-Entfernungseinrichtung (6) mittels einer Tailgasleitung (19) mit dem Kraftwerk (17) und/oder mit dem Reduktionsgasofen (14) verbunden ist.
